Europäisches Patentamt

European Patent Office

Office européen des brevets

(11) Publication number: **0 164 849**

**A1**

# EUROPEAN PATENT APPLICATION

(21) Application number: **85302815.7**

(22) Date of filing: **23.04.85**

(51) Int. Cl.⁴: **G 02 B 6/06**
**A 61 B 1/00**

(30) Priority: **04.05.84 US 607043**

(43) Date of publication of application:
**18.12.85 Bulletin 85/51**

(84) Designated Contracting States:
**DE FR GB**

(71) Applicant: **Warner-Lambert Technologies, Inc.**
**122 Charlton Street**
**Southbridge Massachusetts 01550(US)**

(72) Inventor: **Brunell, Robert A.**
**4 Bellevue Avenue**
**Southbridge Massachusetts 01550(US)**

(74) Representative: **Coxon, Philip et al,**
**Eric Potter & Clarkson 14 Oxford Street**
**Nottingham NG1 5BP(GB)**

(54) Bi-image fibrescope.

(57) A bi-image fibrescope having a primary (16) and a secondary (17) fibre optics image-transmitting bundle. The bundles are joined at the distal ends in a common housing (11) and are separated at the proximate ends to facilitate dual viewing of a remote object by two individuals or by an individual and a camera simultaneously.

FIG.1

## BI-IMAGE FIBRESCOPE

The present invention relates to fibrescopes and relates in particular to fibrescopes that provide at least two independent image-transmitting systems.

Stated otherwise, the invention relates to two parallel flexible image systems joined at the distal ends and separated at the proximate ends so that an object common to both systems can be observed by two individuals or by an individual and a camera simultaneously.

Prior art fibrescopes offer two observation points for simultaneous image observation but do so at a disadvantage and inefficiently. The prior art fibrescopes have a single image-transmitting system and at a point along the image-transmission line a second fibre optic image system is "patched" into the main system. This results in loss of light to the viewer in the main system and loss of light and loss of fidelity to the viewer observing through the second system.

These disadvantages are in addition to the difficulty in connecting a second fibre optic system in series with the main system which frequently results in a moire or ripple pattern or a fuzzy fringe upon the image observed in the second system.

An object of the present invention is to provide a bi-image fibrescope which affords at least two images of high fidelity and of equal quality to two observers or to an observer and a camera.

According to the present invention we provide a bi-image fibrescope comprising a primary image-transmitting fibre optic bundle having a predetermined length, a secondary image-transmitting fibre optic bundle having a second predetermined length, said bundles having a common housing for a first portion of their respective lengths, each bundle having a separate and individual housing for the remainder of their respective lengths and individual proximate ends of each image-transmitting bundle so that two images of the same object can be obtained simultaneously.

Both fibre optic bundles may terminate at their proximate ends in a viewing eyepiece whereby two observers can view the same image simultaneously. Alternatively, one eyepiece can be replaced by or provided with a camera for simultaneous viewing and photographing an image.

Reference is now made to the accompanying drawings in which:-

Figure 1 is a side elevation of a fibrescope embracing the present invention; and

Figure 2 is a schematic end view of the distal end of the fibrescope shaft or common housing.

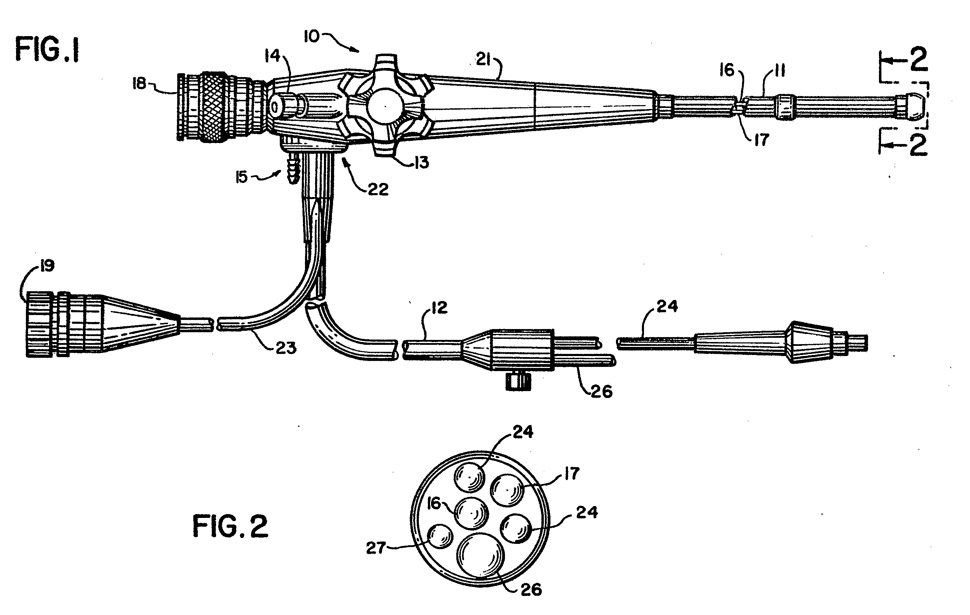

Referring to Figures 1 and 2, the reference numeral 10 designates a fibrescope having a shaft 11, an air and light guide umbilical 12 and a shaft articulating knob 13, a utility entry 14 and a vacuum attachment 15. The utility entry can be used for any conventional purpose such as a biopsy instrument. These items are usual and customary in fibrescopes in the present state of development of the art.

The shaft 11 houses a pair of image-transmitting fibre optic bundles 16 and 17, each of a predetermined length and each terminating in individual eyepieces 18 and 19 respectively. The bundle 16 is termed the primary image transmitter and the bundle 17 the secondary transmitter.

The bundles 16 and 17 are disposed in a generally parallel position in relatively close proximity to one another at the distal end and for a portion of their lengths are housed commonly in shaft 11 and in contiguous scope body 21.

At a point indicated generally by the reference numeral 22, the secondary bundle 17 branches off the scope body 21, housed in a protective umbilical 23, and terminates in a secondary eyepiece 19.

The primary bundle terminates in the usual fashion at a primary eyepiece 18.

Since both bundles are individual and arranged in parallel, in contrast to a series connection, a principal observer at the primary eyepiece 18 has assurance that an associate or a trainee observer at the secondary eyepiece 19 will have the same light and the same fidelity of image that he enjoys.

Furthermore, since the bundles are positioned very close to one another at the distal end, parallax is minimised.

As is most apparent in Figure 2, one or more light guides 24-24 are provided to add illumination to the observed object.

The channel 26 is available for air or vacuum and channel 27 is available for irrigation or other typical utilities.

As stated earlier, the present invention presents the opportunity for two individuals to observe an object simultaneously under substantially the same conditions of image quality and light.

In addition, it is possible to make a camera attachment (not shown) to one bundle, making it possible to photograph an object while observing the object through the eyepiece of the other bundle.

0164849

-4-

CLAIMS

1. A bi-image fibrescope comprising a primary image-transmitting fibre optic bundle having a predetermined length, a secondary image-transmitting fibre optic bundle having a second predetermined length, said bundles having a common housing for a first portion of their respective lengths, each bundle having a separate and individual housing for the remainder of their respective lengths and individual proximate ends of each image-transmitting bundle so that two images of the same object can be obtained simultaneously.

2. A fibrescope according to Claim 1 in which each proximate end of each bundle is provided with a viewing eyepiece.

3. A fibrescope according to Claim 1 or 2 in which the portions of the image-transmitting bundles within the common housing are disposed generally parallel to one another and in close proximity to minimise parallax.

4. A fibrescope according to any of Claims 1 to 3 in which the common housing encloses at least one light-transmitting fibre optic bundle.

5. A fibrescope according to any of Claims 1 to 4 in which one of the proximate ends is fitted with a camera attachment and one with an eyepiece so that an observer can view an object through the eyepiece while a camera is in position.

6. A fibrescope according to any of Claims 1 to 5 which includes an umbilical housing connected to the common housing to provide a source of air, vacuum and/or liquid at the distal end of the fibrescope.

0164849

FIG.1

FIG.2

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| Y | US-A-3 556 085 (NAGASHIGE TAKAHASHI) <br> * Column 6, lines 9-68; figures 4-6 * | 1-4 | G 02 B 6/06 <br> A 61 B 1/00 |
| Y | DE-A-2 801 441 (OLYMPUS OPTICAL CO.) <br> * Claims 1,2; page 6, lines 11-23; page 7, lines 11-38; figure 1 * | 1-4 | |
| A | US-A-3 889 662 (KAZUHIKO MITSUI) <br> * Column 4, lines 4-51; figure 5 * | 1-4 | |
| A | GB-A-1 268 855 (NIPPON SELFOC K.K.) <br> * Page 4, lines 45-113; figure 5 * | 2-4 | TECHNICAL FIELDS SEARCHED (Int. Cl.4) |
| A | DE-A-2 849 729 (K. STORZ) <br> * Page 6, line 22 - page 7, line 25; page 8, line 21 - page 9, line 23; figures 1,3,4 * | 2-5 | G 02 B <br> A 61 B |
| A | GB-A-1 477 070 (FUJI PHOTO OPTICAL CO.) <br> * Page 1, lines 18-32,64-84 * | 6 | |

The present search report has been drawn up for all claims

| Place of search | Date of completion of the search | Examiner |
|---|---|---|
| THE HAGUE | 29-07-1985 | KEMSLEY E.E.K. |